# EUROPÄISCHE PATENTSCHRIFT

(11) **EP 3 136 990 B1**
(45) Veröffentlichungstag und Bekanntmachung des Hinweises auf die Patenterteilung: **13.05.2020**
(21) Anmeldenummer: 15718229.6
(22) Anmeldetag: 24.04.2015
(51) Int. Cl.: A61B 17/128, A61B 17/29

(54) **MAULTEIL FÜR EIN CHIRURGISCHES ROHRSCHAFT-INSTRUMENT**
JAW FOR A SURGICAL TUBULAR SHAFT INSTRUMENT
PARTIE MÂCHOIRE POUR UN INSTRUMENT CHIRURGICAL À TIGE TUBULAIRE

(30) Priorität: 28.04.2014 DE 102014207900
(43) Veröffentlichungstag der Anmeldung: 08.03.2017
(73) Patentinhaber: Aesculap AG, 78532 Tuttlingen (DE)
(72) Erfinder: SCHOLTEN, Thomas, 78532 Tuttlingen (DE); WANKE, Gunnar, 8280 Kreuzlingen (CH)
(74) Vertreter: Winter, Brandl, Fürniss, Hübner, Röss, Kaiser, Polte - Partnerschaft mbB
(86) Internationale Anmeldenummer: PCT/EP2015/058948
(87) Internationale Veröffentlichungsnummer: WO 2015/165822

(56) Entgegenhaltungen:
- EP-A1- 0 945 105
- WO-A2-2006/042110
- WO-A2-2006/042141

## Beschreibung

Die vorliegende Erfindung betrifft ein Maulteil für ein chirurgisches Rohrschaft-Instrument. Ein chirurgisches Rohrschaft-Instrument ist beispielsweise ein endoskopisches Rohrschaft-Instrument zum Applizieren von chirurgischen Clips.

### Stand der Technik

Im Stand der Technik sind einige Maulteile für chirurgische Rohrschaft-Instrumente bekannt. So ist in der europäischen Patentanmeldung EP 1 712 187 A2 und in der Patentanmeldung WO 2006/042141 A2 beispielsweise ein Maulteil gezeigt, bei dem die beiden Branchen elastisch federnd über eine gemeinsame Basis verbunden sind. im Bereich ihrer distalen Enden, welche dazu vorgesehen sind, den chirurgischen Clip zu halten und zusammen zu drücken und den Clip dadurch zu applizieren, weisen die beiden Branchen auf ihren Außenseiten je eine Gleitfläche auf. Um das Maulteil zu schließen und so den Clip zu applizieren, wird das Maulteil gegenüber dem Schaft, in dem es angeordnet ist, nach proximal verschoben (das Maulteil wird also teilweise in den Schaft hinein gezogen bzw. der Schaft wird über das Maulteil geschoben) und der distale Rand des Schafts gleitet an den Gleitflächen ab. Durch die Schrägstellung der Gleitflächen gegenüber der Achse des Schafts werden die distalen Enden der Branchen nach innen gedrängt, während die proximalen Enden der Branchen durch die Basis gehalten werden. Auf diese Weise führen die Branchen jeweils eine Drehbewegung um den Punkt herum aus, an dem die Branchen mit der Basis verbunden sind. Ein Öffnungsvorgang des Maulteils erfolgt zudem ohne Führung und wird ausschließlich durch die Elastizität der Branchen gewährleistet, die in ihre Ausgangsposition zurück drängen, wenn das Maulteil während des Öffnungsvorgangs aus dem Schaft heraus geschoben wird.

Ein vergleichbares Maulteil ist auch in der internationalen Patentanmeldung WO 2008/127 968 gezeigt, auch wenn sich das dort gezeigte Instrument insgesamt stark von dem vorstehend beschriebenen Instrument unterscheidet.

Noch deutlicher wird die Drehbewegung der Branchen beim Öffnen und Schließen des Maulteils aus der US Patentanmeldung US 2005/0171560 A1. Dort sind die distalen Bereiche beider Branchen an der Basis gelenkig angebracht und drehen sich um den Befestigungspunkt. Auch bei dieser Konstruktion wird der Clip appliziert, indem der distale Rand des Schafts an den Gleitflächen, welche an den Außenseiten der Branchen vorgesehen sind, abgleitet und so die Branchen nach innen drängt.

Das Problem dieser Art von Maulteilen ist, dass sie eine immer gleiche Schließgeometrie aufweisen, genauer gesagt, dass sich immer zuerst die distalen Enden der Branchen berühren bzw. aneinander vorbei gleiten und sich der Kontakt bzw. das aneinander vorbei Gleiten der weiter proximal liegenden Bereiche der Branchen dem anschließt. Bei Clipapplikatoren bedeutet dies, dass der Clip immer vom distalen Ende her geschlossen wird. Für andere chirurgische Instrumente wie beispielsweise eine endoskopische Schere ist dieser Aufbau eines Maulteils aus diesem Grund nicht brauchbar.

Ein weiteres Problem dieser Art von Maulteilen ist, dass das Öffnen des Maulteils alleine durch die Elastizität der Branchen realisiert wird. Die Öffnungsbewegung des Maulteils erfolgt ohne Führung. Sollte ein Gewebestück oder ein anderes Teil zwischen den vorderen Rand des Schaftes und eine Branche des Maulteils gelangen, könnte dies den Öffnungsvorgang des Maulteils behindern. Dann müsste das Instrument erst aus dem Hohlraum im Inneren des Patienten entnommen werden, um von dem Gewebestück befreit zu werden, um anschließend wieder in den Patienten eingeführt zu werden. Dies führt zu Verzögerungen und Störungen im Operationsablauf.

EP 0 945 105 A1 offenbart ein Mauteil mit schwenkbare Branchen, die durch einen Schiebemechanismus innerhalb einer Hülse betätigt werden, wobei der Steuermechanismus umfasst eine zylindrische Zahnstange, die mit zwei oder mehr Zähnen in einem runden Abschnitt jeder Branche angrenzend an ihren Drehpunkt eingreift.

### Aufgabe der Erfindung

Die Aufgabe der vorliegenden Erfindung ist es, ein Maulteil für ein chirurgisches Rohrschaft-Instrument bereit zustellen, bei dem zum einen die Schließgeometrie des Maulteils beliebig einstellbar ist und bei dem zum anderen ein geführtes Schließen und Öffnen des Maulteils erfolgt. Die Aufgabe der vorliegenden Erfindung wird durch ein Maulteil für ein chirurgisches Rohrschaft-Instrument gemäß dem Anspruch 1 der vorliegenden Anmeldung gelöst. Vorteilhafte Weiterbildungen und Ausgestaltungen des Maulteils sind Gegenstand der abhängigen Ansprüche. Begriffsdefinitionen

Der Begriff des chirurgischen Rohrschaft-Instruments umfasst in dieser Anmeldung zum einen endoskopische Instrumente wie beispielsweise endoskopische Clipapplikatoren oder Nadelhalter. Zum anderen umfasst dieser Begriff aber auch chirurgische Instrumente für eine offene Operation, bei denen der Funktionsabschnitt bzw. der Wirkabschnitt des Instruments durch einen Schaft oder ein schaftartiges Bauteil von dem Betätigungsabschnitt bzw. dem Griffabschnitt getrennt ist. Der Begriff Schaft bzw. schaftartiges Bauteil bezeichnet hierbei ein Bauteil, dessen Abmessungen und Lage gegenüber dem Betätigungsabschnittl (z.B. Griffstück) auch während einer Betätigung des chirurgischen Instruments im Wesentlichen unveränderlich ist. Eine axiale Verschiebung entlang der Achse des Schafts oder schaftartigen Bauteils bzw. eine Verdrehung um diese Achse herum ist dabei zulässig, nicht jedoch eine wesentliche Verschiebung quer zu dieser Achse oder eine Verdrehung gegenüber dieser Achse in der Weise, dass sich die beiden Enden des Bauteils wesentlich von dieser Achse entfernen. Vorzugsweise ist die Länge eines Schafts oder schaftartigen Bauteils größer als dessen beide anderen Abmessungen (Breite, Tiefe) und es ist weiter vorzugsweise schlank ausgebildet. Der Schaft bzw. das schaftartige Bauteil muss dabei nicht rund, geschlossen, rohrförmig oder dünnwandig sein. Entscheidend ist hierbei, dass es sich um ein Instrument handelt, welches nicht wie eine gewöhnliche Schere einen Drehpunkt aufweist, um welchen sich alle wesentlichen Bestandteile des Instruments drehen, sondern dass die Kraft zum Öffnen und Schließen des Maulteils über eine relative axiale Bewegung eines Bauteils gegenüber dem Schaft übertragen wird.

Der Funktionsabschnitt bzw. Wirkabschnitt ist in dieser Anmeldung der Bereich des chirurgischen Rohrschaft-Instruments, an dem dessen eigentliche Funktion ausgeführt wird. Bei einem Nadelhalter ist es der Bereich, der die Nadel greift und hält, also die distalen Bereiche der Branchen. Bei einer Schere ist es der Bereich, der das Gewebe oder etwas anderes durchtrennt, also der Bereich, an dem die beiden aneinander vorbei gleitenden Scherkanten ausgebildet sind. Bei einem Clipapplikator ist es der Bereich, in dem der Clip zunächst gehalten wird, währen er durch den Chirurgen an die richtige Stelle und in die richtige Lage gebracht wird, und in dem der Clip anschließend appliziert wird, d.h. verpresst wird. Bei anderen Instrumenten ist die Definition des Funktionsabschnitts bzw. Wirkabschnitts entsprechend anzuwenden.

Der Wirkbereich ist der Bereich einer einzelnen Branche, an dem diese die bestimmungsgemäße Funktion des Instruments bewirkt, also bei einem Nadelhalter ein Greifbereich, bei einer Schere eine Scherkante und bei einem Clipapplikator ein Anlagebereich des Clips.

### Allgemeine Beschreibung der Erfindung

Die vorliegende Erfindung ist durch die Merkmale des unabhängigen Anspruchs definiert. Bevorzugte Ausführungsformen sind in den abhängigen Ansprüchen angegeben.

Gemäß der vorliegenden Erfindung weist ein Maulteil für ein chirurgisches Rohrschaft-Instrument ein Haltebauteil, eine erste Branche und einer zweite Branche auf. Ein chirurgisches Rohrschaft-Instrument bezeichnet hierbei nicht nur ein endoskopisches chirurgisches Instrument sondern auch ein chirurgisches Instrument für die offene Chirurgie. Bei dem erfindungsgemäßen Maulteil verfügt die erste Branche und die zweite Branche über je ein Kulissenelement und die Branchen sind durch das Haltebauteil in axialer Richtung gehalten. Wenn nur eine Branche über ein Kulissenelement verfügt, ist die andere Branche zumeist relativ zu dem Schaft des chirurgischen Instruments fixiert, sodass eine Branche unbeweglich ist und sich die andere Branche auf diese unbewegliche Branche zu und/oder an dieser vorbei und von dieser weg bewegt, wenn das Maulteil geschlossen und geöffnet wird. Darüber hinaus weist das Maulteil ein Nockenträgerbauteil auf, das relativ zu dem Haltebauteil in axialer Richtung verschiebbar ist und mindestens zwei Nocken trägt. Jedes Kulissenelement ist zudem daran angepasst, bei einer relativen axialen Verschiebung zwischen dem Haltebauteil und dem Nockenträgerbauteil mit mindestens zwei Nocken, die an Nockenträgerbauteil vorgesehen sind, in Kontakt zu stehen und an diesen abzugleiten, um so ein Öffnen bzw. Schließen des Maulteils zu bewirken.

Mit Hilfe des erfindungsgemäßen Maulteils ist ein gesteuertes Öffnen und Schließen des Maulteils möglich. So kann es auch sein, dass sich die Wirkbereiche bei einem Schließvorgang zu Beginn oder während des Schließvorganges zeitweise voneinander entfernen und somit eine Aufweitung des Maulteils bewirken, also eine Vergrößerung des Abstands der beiden Wirkbereiche zueinander. Der Schließvorgang muss somit keine stetige Bewegung in eine Richtung sein, wichtig ist lediglich, dass während des Schließvorgangs die Aufgabe ausgeführt wird, die dem jeweiligen Maulteil bestimmungsgemäß zugewiesen ist. So appliziert ein Clipapplikator einen Clip, indem das Maulteil geschlossen wird, eine Schere schneidet z.B. das Gewebe und eine Zange greift das zu greifende Objekt ebenfalls während des Schließvorgangs. Ein Öffnen entspricht dem entgegengesetzten Vorgang und somit z.B. dem Loslassen des Gewebes durch die Zange bzw. die Wirkbereiche der Branchen der Zange.

Mit dem erfindungsgemäßen Maulteil kann die Öffnungs- und Schließkinematik des Maulteils eingestellt und so an die jeweilige Aufgabe des Maulteils angepasst werden. So kann zum Beispiel bei einem Clipapplikator bei einem Schließvorgang das Maulteil zunächst ein wenig geöffnet werden, d.h. die Wirkbereiche der Branchen entfernen sich zunächst voneinander. Bei der Verwendung eines Clips mit einer gewissen Elastizität, der unter einer gewissen Vorspannung in das Maulteil eingebracht ist, wenn es sich in der Ausgangsposition befindet, weitet sich der Clip dann zunächst ein wenig auf und folgt somit der Öffnung des Maulteils, sodass er nicht aus dem Maulteil fällt. Anschließend werden die Wirkbereiche der Branchen z.B. zunächst recht schnell aufeinander zu bewegt und zum Ende des Schließvorgangs, wenn der Clip das zu verschließende Gefäß bereits recht eng umschließt und eine erhöhte Verpresskraft erforderlich wird, um den Clip vollständig zu schließen, werden die Wirkbereiche der Branchen langsamer als zuvor aufeinander zu bewegt, um so ein hinreichendes Verpressen des Clips und somit einen guten Verschluss des Gefäßes sicher zu stellen. Bei herkömmlichen Maulteilen ist eine Aufweitung während des Schließvorgangs nicht möglich, da die herkömmlichen Maulteile geschlossen werden, indem von lateral außen auf die beiden elastisch verbundenen Branchen gedrückt wird. Mit einem solchen Aufbau ist ein Aufweiten des Maulteils nicht erzielbar. Aber auch ein gesteuerter Öffnungsvorgang bietet Vorteile, die durch die herkömmlichen Maulteile nicht erzielbar sind. Ist das Maulteil eines herkömmlichen Instruments geschlossen und hat sich ein Gewebestück zwischen die Branchen geklemmt, kann es vorkommen, dass sich das Maulteil nicht öffnet, wenn der Chirurg den Betätigungsabschnitt des chirurgischen Instruments frei gibt. Dies liegt daran, dass die Öffnungsbewegung des Maulteils nur durch die Elastizität der Branchen erreicht wird. Diese Elastizität ist aber zumeist relativ gering, da eine hohe Elastizität während des Schließvorgangs hinderlich wäre. Insbesondere bei endoskopischen Instrumenten sind die Platzverhältnisse im Instrument extrem beengt und alle Bauteile werden so schlank wie möglich ausgebildet. Aus diesem Grund würde eine erhöhte Federsteifigkeit der Befestigungsabschnitte der Branchen der Maulteile zu einem vergrößerten Querschnitt derselben führen. Geleichzeitig müsste das Bauteil im Querschnitt vergrößert werden, welches die Schließkraft auf die Branchen ausübt. Dies würde entweder zu einem vergrößerten Gesamtquerschnitt des Maulteils führen oder aber zu einer verringerten Belastbarkeit desselben bei nicht angepassten Querschnitten. Wenn sich ein solches Maulteil also einmal nicht mehr öffnet, muss das Instrument aus dem Operationsbereich entnommen, durch z.B. den Chirurgen manuell am Maulteil geöffnet und anschließend wieder an/in den Operationsbereich eingeführt werden. Dies führt nicht nur zu einer Verzögerung im Operationsablauf sondern bedeutet auch eine potentielle Gefahr für den Patienten, einerseits dadurch, dass eine Blutung womöglich nicht schnell genug gestillt werden kann, andererseits durch den möglichen Eintrag von Verschmutzungen und Krankheitserregern durch das Manipulieren am Maulteil des Instruments und das anschließende Wiedereinführen in den Patienten. Es besteht aber auch eine Verletzungsgefahr für die Person, die das Maulteil wieder gängig macht, insbesondere wenn es sich bei dem Instrument um eine Schere oder ein anderes Instrument mit schaften Kanten handelt.

Mit einem erfindungsgemäßen Maulteil mit gesteuerter Öffnungs- und Schließkinematik stellt sich dieses Problem nicht. Sollte sich einmal z.B. ein Stück Gewebe zwischen den beiden Branchen des Maulteils oder zwischen einer Branche und dem Schaftbauteil verklemmen, kann über die Ansteuerung der einen Branche oder der beiden Branchen über die Nocken und Kulissen ausreichend Kraft aufgebracht werden, um das Maulteil zu öffnen, ohne es aus dem Operationsgebiet heraus nehmen zu müssen. Auf diese Weise entsteht keine Verzögerung im Operationsablauf und die vorstehend beschriebenen Gefahren für Patienten und Operateur entfallen. Gemäß einer vorteilhaften Ausführungsform der vorliegenden Erfindung ist das Haltebauteil einstückig mit einem Schaftbauteil des Schafts ausgebildet oder es ist an diesem befestigt. Das Nockenträgerbauteil ist zudem ein Schieber, welcher relativ zu dem Instrumentenschaft axial bewegbar ist. Dies entspricht einem besonders vorteilhaften Aufbau, da der Schieber im Inneren des Schafts bzw. des Schaftbauteils angeordnet werden kann. Die Maulteile müssen sich somit nicht gegenüber dem Schaft bzw. dem Schaftbauteil in axialer Richtung bewegen, was es dem Chirurgen erleichtert, das Maulteil exakt zu positionieren und zu bedienen.

Gemäß einer weiteren vorteilhaften Ausführungsform der vorliegenden Erfindung sind die erste Branche und die zweite Branche elastisch gekoppelt. Bei einer elastischen Kopplung der beiden Branchen, bevorzugt an deren proximalem Bereich, kann insbesondere der Zusammenbau des Maulteils erleichtert werden, da weniger einzelne Bauteile vorliegen.

Gemäß einer besonders vorteilhaften Ausführungsform der vorliegenden Erfindung sind an mindestens einem Kulissenelement mindestens zwei Kulissenbahnen ausgebildet. Mit dieser Ausgestaltung ist es möglich, verschiedenartigste Öffnungs- und Schließkinematiken des Maulteils zu verwirklichen. Zudem kann, wenn die Nocken an verschiedenen Kulissenbahnen entlang gleiten, deren Abstand zueinander leicht vergrößert werden, sodass ein größeres Moment auf den Wirkbereich der jeweiligen Branche aufgebracht werden kann.

Gemäß einer vorteilhaften Ausführungsform der vorliegenden Erfindung verfügt die erste Branche und/ oder die zweite Branche über wenigstens einen Vorsprung, der in einen Bereich des Haltebauteils eingreift und so eine axiale Bewegung der Branche gegenüber dem Haltebauteil begrenzt und bevorzugt verhindert. Diese Ausgestaltung ermöglicht eine besonders leichte axiale Anbindung der jeweiligen Branche an das Haltebauteil. Wenn das Haltebauteil den Vorsprung in axialer Richtung des Maulteils formschlüssig aufnimmt, ist die jeweilige Branche gegenüber dem Haltebauteil axial fixiert. Ist der Vorsprung z.B. in einer Art Langloch aufgenommen, ist die axiale Bewegung der Branche gegenüber dem Haltebauteil begrenzt aber nicht vollständig fixiert. Wichtig ist hierbei, dass der Weg, den sich die Branche gegenüber dem Haltebauteil bewegen kann, geringer ist als der Weg, den sich das Nockenträgerbauteil degenüber dem Haltebauteil bewegt, da es ansonsten passieren kann, dass keine Bewegung zwischen Nockenträgerbauteil und Haltebauteil erfolgt und somit kein Öffnungs- und Schließvorgang erzeugt werden kann. Vorzugsweise bewegt sich die jeweilige Branche in axialer Richtung im Wesentlichen nicht gegenüber dem Haltebauteil.

Gemäß einer anderen vorteilhaften Ausführungsform der vorliegenden Erfindung ist der wenigstens eine Vorsprung an einem biegeelastischen Fortsatz des zugehörigen Kulissenelements der Branche vorgesehen und der biegeelastische Fortsatz drängt den Vorsprung zu dem Haltebauteil hin und sichert so einen Eingriff des wenigstens einen Vorsprungs in das Haltebauteil, wobei die Biegeelastizität des Fortsatzes bevorzugt derart eingestellt ist, dass die Bewegbarkeit und die Bewegungen des Kulissenelements durch den Fortsatz im Wesentlichen nicht beeinflusst sind. Vorteilhafter Weise ist der mindestens eine Vorsprung der jeweiligen Branche an einem elastischen Fortsatz angeordnet, der sich von der Branche in proximaler Richtung, also von dem Wirkbereich der Branche weg, erstreckt. Dieser Fortsatz drängt durch seine Biegeelastizität bevorzugt seitlich gegen das Haltebauteil und drückt den Vorsprung in eine Aufnahme in dem Haltebauteil. Die Biegeelastizität sichert den Vorsprung somit vor einem Herausrutschen oder Herausspringen aus der Aufnahme. Weiter vorzugsweise verfügt die jeweilige Branche über zwei Vorsprünge, welche in axialer Richtung des Maulteils hintereinander angeordnet sind. Auf diese Weise verteilt sich die Haltekraft, welche von jedem Vorsprung und der entsprechenden Gegenfläche der Aufnahme an dem Haltebauteil übertragen bzw. aufgenommen werden muss. Weiter vorzugsweise sind die Abstände zwischen den Kraftübertragungsflächen der beiden Vorsprünge ein wenig geringer als der Abstand der entsprechenden Gegenflächen am Haltebauteil. Wenn das Nockenträgerbauteil bei einem solchen Aufbau nach distal verschoben wird, um einen Schließvorgang des Maulteils zu bewirken, wird die jeweilige Branche im Rahmen ihres Spiels auch nach distal verschoben. Wenn nun zuerst der proximalere Vorsprung eine Haltekraft überträgt, bewirkt dies ein Moment in dem elastischen Fortsatz, welcher den distaleren Vorsprung gegen das Haltebauteil drängt. Kurz darauf überträgt auch der distalere Vorsprung einen Teil der Haltekraft, sodass sich die Branche nicht wesentlich in axialer Richtung bewegen kann. Mit dieser Anordnung ist aber der distalere Vorsprung doppelt gegen ein Herausrutschen oder Herausspringen aus der Aufnahme in dem Haltebauteil gesichert.

Gemäß noch einer vorteilhaften Ausführungsform der vorliegenden Erfindung ist mindestens ein Kulissenelement im Wesentlichen flächig ausgebildet, das Nockenträgerbauteil ist im Wesentlichen auch flächig ausgebildet und das mindestens eine Kulissenelement liegt im Wesentlichen so an einer flachen Seite des Nockenträgerbauteils an, dass ein sandwichartiger Aufbau ausgebildet ist. Bevorzugt ist je ein Kulissenelement auf beiden Seiten des Nockenträgerbauteils angeordnet, d.h. das Nockenträgerbauteil ist zwischen den beiden Kulissenelementen angeordnet. Auf diese Weise kann ein Maulteil ausgebildet werden, das außerhalb des Bereichs, in dem die Wirkflächen angeordnet sind, einen sehr flachen Aufbau aufweist. Auf diese Weise ist ein Zugang zu dem Wirkbereich aus proximaler Richtung möglich, sodass von dort bei einem Clipapplikator beispielsweise Clips in das Maulteil zugeführt werden können.

Gemäß noch einer vorteilhaften Ausführungsform der vorliegenden Erfindung bilden das Nockenträgerbauteil und mindestens ein Kulissenelement mindestens einen Bereich aus, in dem eine Kulissenbahn und die zugehörige Nocke des Nockenträgerbauteils eine Hinterschneidung ausbilden, sodass ein Abheben des Kulissenelements von dem Nockenträgerbauteil verhindert ist. Wenn die Ebene, in der das Maulteil seine Arbeit verrichtet, nicht mit der Ebene identisch ist, in der die Nocken des Nockenträgerbauteils an der Kulisse der jeweiligen Branche anliegt, entsteht ein Moment, welche ein Abheben des Kulissenelements von dem Nockenträgerbauteil bewirkt. Die Hinterschneidung zwischen Nocke und Kulissenbahn verhindert aber dieses Abheben, sodass der sandwichartige Aufbau von Nockenträgerbauteil und Kulissenelement erhalten bleibt. Bevorzugt ist mindestens ein Bereich einer Hinterschneidung über den gesamten Bewegungsbereich des Kulissenelements zu dem Nockenträger von einer vollständig geöffneten Stellung zu einer vollständig geschlossenen Stellung des Maulteils vorhanden. Weiter bevorzugt gibt es eine Montagestellung, welche sich außerhalb des Bereichs von der vollständig geöffneten Stellung zu der vollständig geschlossenen Stellung des Maulteils befindet, in der diese Hinterschneidung aufgehoben werden kann. Die Montagestellung kann von dem Maulteil vorzugsweise nicht mehr eingenommen werden, nachdem es an einem Schaft bzw. Schaftbauteil eines chirurgischen Instruments montiert wurde. Wenn keine Hinterscheidung zwischen Nockenträgerbauteil und Kulissenelement vorgesehen ist, kann ein Bauteil vorgesehen sein, welche die jeweiligen Bauelemente vor einem gegenseitigen Abheben sichert, beispielsweise eine Klammer.

Gemäß einer besonders vorteilhaften Ausführungsform der vorliegenden Erfindung ist das chirurgische Instrument ein chirurgischer Clipapplikator und die Branchen des Maulteils sind daran angepasst, einen chirurgischen Clip zu halten und durch ein Schließen des Maulteils zu applizieren. Vorzugsweise wird der Clip von den Wirkbereichen der Branchen gehalten und appliziert. Weiter vorzugsweise ist der chirurgische Clip ein Doppelstegclip, der insbesondere aus zwei Cliphälften besteht, die jeweils nur an ihren beiden distalen Enden miteinander verbunden sind. Die steuerbare Öffnungs- und Schließkinematik, welche vorstehend beschreiben ist, eignet sich insbesondere für chirurgische Clipapplikatoren, da es mit einer solchen Öffnungs- und Schließkinematik möglich ist, Clips mit einem Applikator zu applizieren, deren maximale laterale Abmessung an ihrem distalen Ende größer als der Durchmesser des Maulteils in der Grundstellung ist und auch größer als der Durchmesser des Schafts des Instruments ist.

Gemäß noch einer weiteren vorteilhaften Ausführungsform der vorliegenden Erfindung sind die Branchen des Maulteils daran angepasst, in der vollständig geöffneten Stellung des Maulteils durch einen Clip, der im Maulteil angeordnet ist, nach außen über die laterale Position der Branchen hinaus verschoben zu werden, welche die Branchen in der vollständig geöffneten Stellung des Maulteils einnehmen, wenn kein Clip im Maulteil angeordnet ist. Mit dieser Anordnung sind die Branchen zwischen Kulissenelement und Wirkbereich elastisch ausgebildet und/oder es ist ein gewisses Spiel zwischen wenigstens einer Nocke und dem Kulissenelement vorgesehen. In letzterem Fall wird das Maulteil aber nicht nur durch die gesteuerte Öffnungscharakteristik aufgeweitet, wie dies vorstehend beschrieben ist, sondern die Elastizität des Clips ist in der Lage, die Wirkbereiche der Branchen auseinander zu drängen.

Gemäß noch einer anderen vorteilhaften Ausführungsform der vorliegenden Erfindung sind an mindestens einem Kulissenelement mindestens drei Kulissenbahnen ausgebildet, wobei zu jedem Zeitpunkt während des Öffnungs- und Schließvorgangs des Maulteils mindestens zwei Kulissenbahnen an jeweils einer Nocke anliegen, welche an dem Nockenträger vorgesehen ist. Mit einem solchen Aufbau kann bei einem Clipapplikator während des Schließvorgangs eine stets definierte Stellung der jeweiligen Branchen gewährleistet sein, indem ein Kontaktpunkt der Branche zum Clip und zwei Kontaktpunkte der Branche zu zwei Nocken vorhanden ist, und während eines Öffnungsvorgangs kann eine stets definierte Stellung der Branche gewährleistet sein, indem drei Kontaktpunkte der Branche zu drei Nocken vorhanden sind. Bei der Schließbewegung können auch drei Kontaktpunkte zwischen der Branche und drei Nocken und zusätzlich ein Kontaktpunkt zwischen Branche und Clip vorliegen. Wenn von Kontaktpunkten zwischen Branche und Nocke die Rede ist, bezieht sich das auf das Kulissenelement der Branche bzw. auf die Kulissenbahnen des Kulissenelements.

Gemäß noch einer vorteilhaften Ausführungsform der vorliegenden Erfindung weist das Maulteil einen vorzugsweise austauschbar anbringbaren Clipspeicher auf, in dem eine Vielzahl von Clips vorgesehen sind, wobei der Clipspeicher zumindest teilweise in einer Ebene angeordnet ist, die parallel zu dem sandwichartigen Aufbau des mindestens einen Kulissenelements mit dem Nockenträgerbauteil ist, wobei die Clips zumindest teilweise in dem Clipspeicher an dem sandwichartigen Schichtaufbau vorbei zu den distalen Bereichen der Branchen zuführbar sind. Mit diesem Aufbau wird ein sogenanntes Mehrschuß-Maulteil bereit gestellt, welches eine Vielzahl von Clips applizieren kann, ohne dass diese Clips manuell einzeln in das Maulteil eingesetzt werden müssen, beispielsweise indem der Clipapplikator von dem Operationsgebiet entnommen, beladen und dann wieder zu dem Operationsgebiet hingeführt werden muss. Im Stand der Technik sind zahlreiche Clipspeicher und Zuführprinzipien bekannt, wie ein Clip automatisch in ein Maulteil eines Clipapplikators eingeführt werden kann.

Gemäß einer vorteilhaften Ausführungsform der vorliegenden Erfindung weisen zumindest die distalen Bereiche der ersten Branche und der zweiten Branche eine zu einer Mittelachse des Maulteils symmetrische Bewegungskurve auf. Auf diese Weise kann sicher gestellt werden, dass die Aufgabe des Maulteils auf dessen Mittelachse ausgeführt wird, d.h. bei einem Clipapplikator wird der Clip exakt mittig zwischen den Wirkbereichen der Branchen appliziert und bei einer Schere wird der Schnitt genau entlang der Mittelachse des Maulteils durchgeführt. Dies verbessert das Handling des Maulteils für den Chirurgen und macht das Behandlungsergebnis leichter vorhersehbar.

Gemäß einer vorteilhaften Ausführungsform der vorliegenden Erfindung ist das chirurgische Instrument eine Schere, ein Nadelhalter, eine Klemme oder ein anderes chirurgisches Instrument, bei dem zwei Branchen aufeinander zu und/oder aneinander vorbei bewegbar sind. Dementsprechend werden vorstehend genannte Instrumente bereit gestellt, die eine genau definierte Öffnungs- und Schließcharakteristik aufweisen und den entsprechenden herkömmlichen Instrumenten klar überlegen sind. Alternativ dazu kann die vorliegende Erfindung auch auf Spreizinstrumente angewendet werden, wobei bei diesen die bestimmmungsgemäße Funktion während des Öffnungsvorgangs und nicht während des Schließvorgangs bewirkt wird.

Erfindungsgemäß wird folglich ein Maulteil mit den vorstehend genannten Merkmalen oder individuell beanspruchbaren Merkmalskombinationen vorgeschlagen, das der Schwenkachsenlosen/Schwenkbolzenlosen Bauart angehört, also keine gegenständliche Schwenkachse aufweist.

Weitere Vorteile und Merkmale der Erfindung sind dem Fachmann aus den beigefügten Figuren und der detaillierten Beschreibung der Ausführungsbeispiele ersichtlich.
- Fig. 1: zeigt ein Maulteil gemäß einem ersten Ausführungsbeispiel der vorliegenden Erfindung;
- Fig. 2: zeigt eine erste Branche des Maulteils nach Fig. 1;
- Fig. 3: zeigt eine zweite Branche des Maulteils nach Fig. 1;
- Fig. 4: zeigt eine Nockenträgerbauteil des Maulteils nach Fig. 1;
- Fig. 5: zeigt eine Maulteil nach Fig. 1 in einem Schaftbauteil eines Instruments, wobei die obere Hälfte des Schaftbauteils freigeschnitten ist;
- Fig. 6: zeigt eine vereinfachte Darstellung der ersten Branche und des Nockenträgerbauteils nach Fig. 1 im Verlauf eines Schließvorgangs;
- Fig. 7: zeigt eine Detailansicht des Maulteils der Fig. 1 von oben, wobei Fig. 7A einen geöffneten und Fig. 7B einen geschlossenen Zustand des Maulteils zeigt;
- Fig. 8: zeigt eine perspektivische Ansicht des Maulteils der Fig. 1, wobei die Fig. 8A eine geöffneten und die Fig. 8B einen geschlossenen Zustand des Maulteils zeigt;

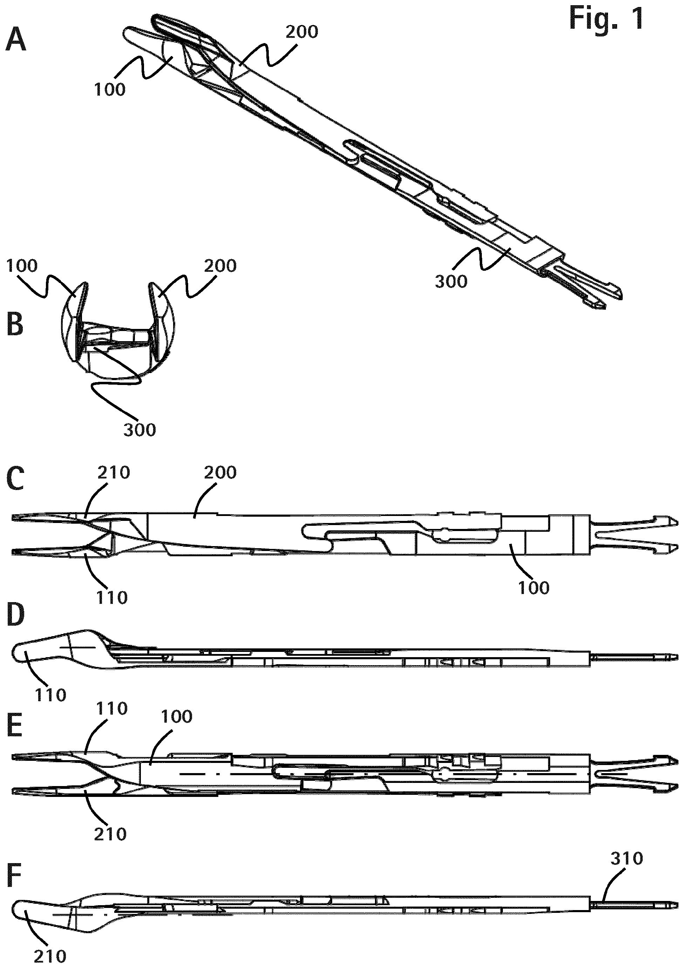

Ein erstes Ausführungsbeispiel der vorliegenden Erfindung ist im Folgenden unter Bezugnahme auf die Fig. 1 bis 5 im Detail beschrieben. Die Fig. 1 bis 5 zeigen jeweils im Figurenteil A eine isometrische Ansicht des Gegenstandes, im Figurenteil B eine Ansicht vom distalen Ende her, im Figurenteil C eine Ansicht von oben, im Figurenteil D eine Ansicht von vorn, im Figurenteil E eine Ansicht von unten und im Figurenteil F eine Ansicht von hinten bezogen auf die isometrische Ansicht.

Das erste Ausführungsbeispiel der vorliegenden Erfindung betrifft einen chirurgischen Clipapplikator und genauer genommen einen chirurgischen Clipapplikator für Doppelstegclips der Mehrschussart (sog. multifire Clipapplikator). Ein Clipapplikator der Mehrschussart verfügt über ein Magazin, in dem Clips gelagert sind und aus dem ein Clip nach der Applikation des im Maulteil befindlichen Clips in dieses mittels eines Zuführmechanismus in das Maulteil und genauer gesagt den Wirkbereich des Maulteils zugeführt wird. Im Gegensatz dazu weist ein Clipapplikator der Einschussart (sog. singlefire Clipapplikator) keinen Zuführmechanismus und kein Magazin auf. Jeder Clip muss dann einzeln manuell in den Wirkbereich des Maulteils des Clipapplikators eingebracht werden. Das Magazin ist auswechselbar an dem Schaft des Clipapplikators montiert. Die bei diesem Ausführungsbeispiel verwendeten Doppelstegclips sind sogenannte Ringclips, also Doppelstegclips, die aus einem Ring geformt werden, welcher aus einem Blech gestanzt wird.

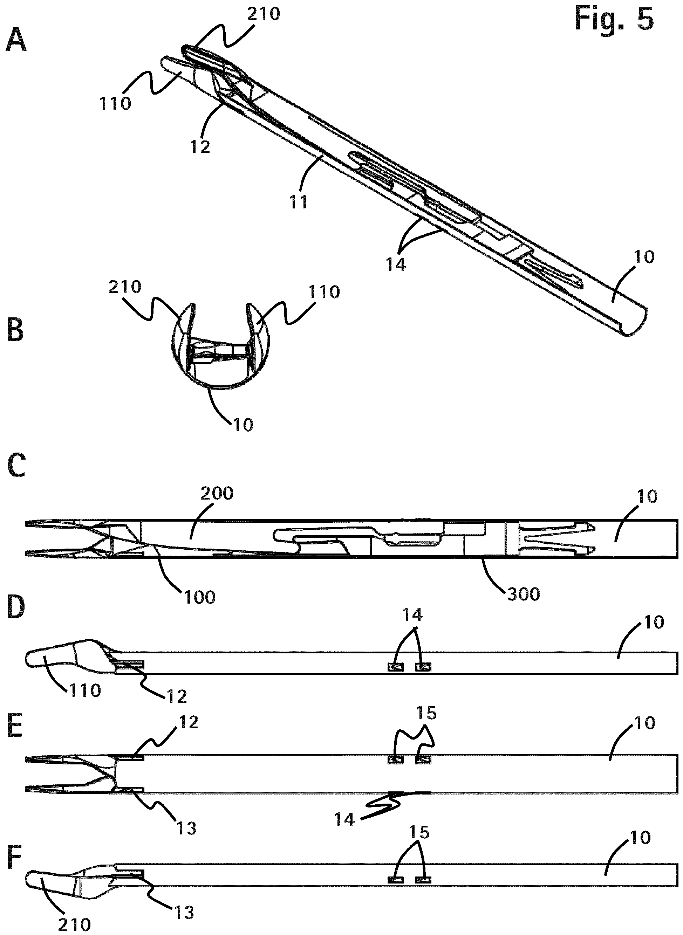

Ein Mehrschuss-Doppelstegclip-Applikatormaulteil 1 gemäß diesem Ausführungsbeispiel weist ein Haltebauteil 10 auf. Das Haltebauteil 10 ist bei diesem Ausführungsbeispiel durch ein Schaftrohr 10 gebildet, von dem in Fig. 5 nur die untere Hälfte dargestellt ist. Das Schaftrohr 10 ist an seinem proximalen Ende mit einem Griff des Clipapplikators lösbar verbindbar. Das Haltebauteil 10 kann aber auch durch ein weiteres Bauteil gebildet sein, welches an einem Schaft bzw. Schaftbauteil eines Applikatorschafts anbringbar ist. In diesem Fall ist das Haltebauteil 10 mittelbar an einem Griff des Applikators anbringbar. An dem distalen Ende 11 des Schaftrohres 10, aus dem Wirkbereiche 110, 210 von Branchen 100, 200 hervorstehen, sind Schlitze 12, 13 vorgesehen. Zudem sind in dem Schaftrohr 10 auf beiden Seiten je zwei Aussparungen 14 und 15 vorgesehen.

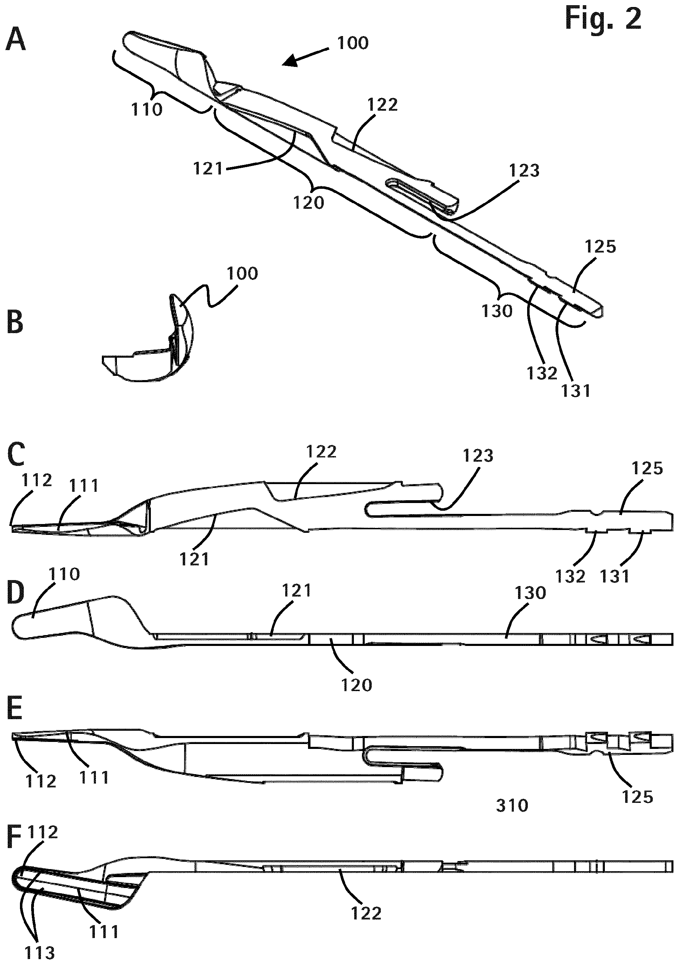

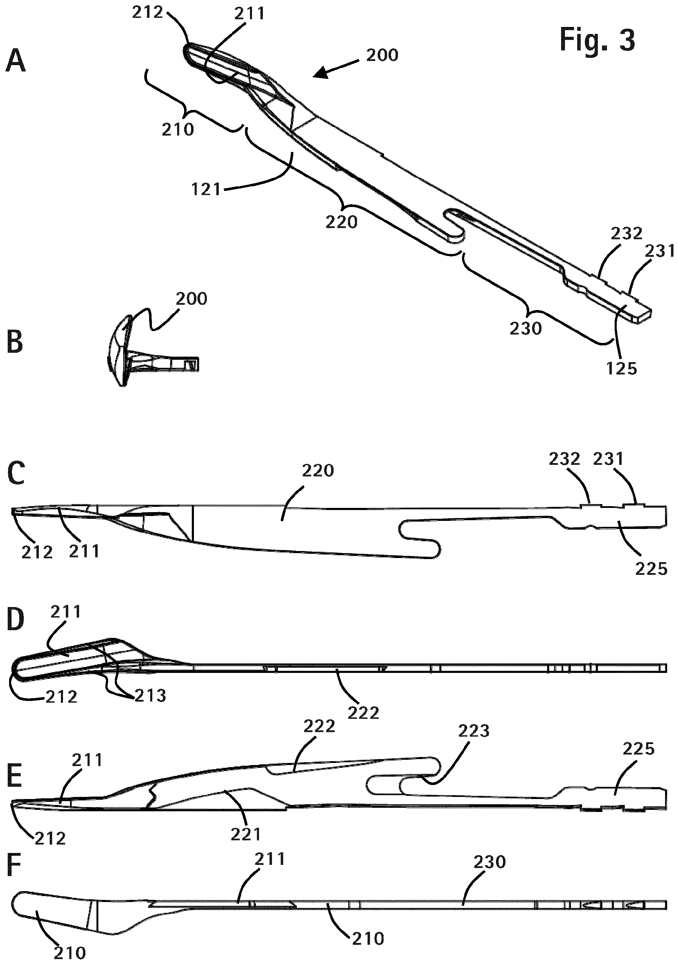

Die erste Branche 100, wie sie in Fig. 2 gezeigt ist, und die zweite Branche 200, wie sie in Fig. 3 gezeigt ist, verfügen, wie dies vorstehend bereits gezeigt ist, über je einen Wirkbereich 110, 210 sowie je ein flächig ausgebildetes Kulissenelement 120, 220 und je einen biegeelastischen Fortsatz 130, 230. Der Wirkbereich 110, 210 jeder Branche 100, 200 weist einen Kanal 111, 211 auf, in dem ein chirurgischer Clip von einem nicht gezeigten Magazin mittels eines ebenfalls nicht gezeigten Zuführmechanismus in den Wirkbereich 110, 210 der Branchen 100, 200 zugeführt werden kann. Damit der Clip nicht nach vorn (distal) aus dem Wirkbereich 110, 210 des Maulteils 1 heraus fallen kann, ist ein Anschlag 112, 212 vorgesehen. Dieser Anschlag 112, 212 ist an die Form des distalen Bereichs des Clips angepasst, sodass der Clip im Wesentlichen entlang seines gesamten distalen Bereichs an dem Anschlag 112, 212 und einer seitlichen Umrandung 113, 213 des Kanals 111, 211 an den Branchen 100, 200 anliegt. Der elastische Fortsatz 130, 230 erstreckt sich von dem Kulissenelement 120, 220 jeder Branche 100, 200 in proximaler Richtung.

Der elastische Fortsatz 130, 230 ist insbesondere in seinem distalen Bereich, also dort, wo er mit dem Kulissenelement 120, 220 verbunden ist, biegeelastisch ausgebildet. Eine Elastizität, die zu einer nennenswerten Verlängerung des Fortsatzes 130, 230 führt, ist bei diesem Ausführungsbeispiel nicht erwünscht. Am proximalen Ende jedes elastischen Fortsatzes 130, 230 sind zwei Vorsprünge 131, 132 bzw. 231, 232 vorgesehen, die von dem jeweiligen elastischen Fortsatz 130, 230 in radialer Richtung vorstehen. Diese Vorsprünge 131, 132 bzw. 231, 232 sind so bemessen, dass sie in die Aussparungen 14, 15 passen, welche in dem Schaftrohr 10 ausgebildet sind. Die elastischen Fortsätze 130, 230 sind zudem so ausgebildet, dass deren proximale Enden durch die Form und Elastizität der Fortsätze gegen die Innenwand des Schaftrohres gedrängt werden, sodass die Vorsprünge 131, 132 bzw. 231, 232 sicher in den Aussparungen 14, 15 aufgenommen sind. Dazu sind die elastischen Fortsätze so ausgebildet, dass deren proximalen Enden leicht nach außen gebogen verlaufen. Die Elastizität der Fortsätze ist dabei so bemessen, dass eine hinreichende Drängkraft in radialer Richtung nach außen vorherrscht, um die Vorsprünge 131, 132 bzw. 231, 231 in den Aussparungen 14, 15 zu sichern, gleichzeitig aber die Bewegung der Kulissenelemente 120, 220 und der Wirkbereiche 110, 210 nicht wesentlich beeinträchtigt bzw. gehemmt wird.

Die Branchen 100, 200 sind durch die Vorsprünge 131, 132 bzw. 231, 232, die in die Aussparungen 14, 15 in dem Schaftbauteil 10 eingreifen, gegenüber dem Schaftbauteil 10 in axialer Richtung im Wesentlichen unverschiebbar gehalten.

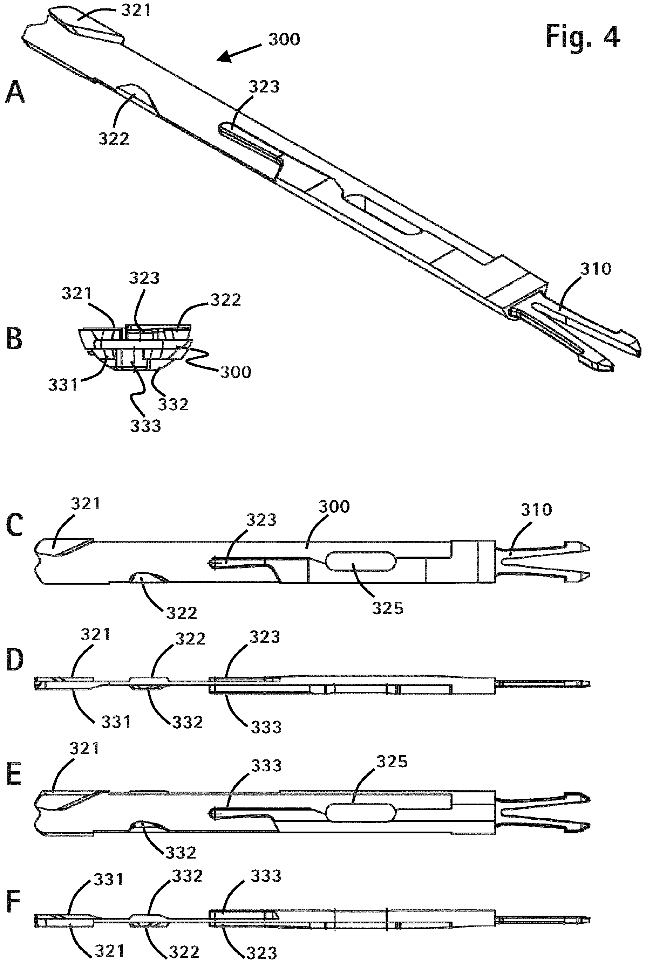

Ein Nockenträgerbauteil 300, wie es in Fig. 4 gezeigt ist und welches im Folgenden Schieber genannt ist, ist zwischen den beiden Kulissenelementen 120, 220 der beiden Branchen 100, 200 angeordnet. Der Schieber 300 ist an seinem proximalen Ende mit einer Befestigungsspange 310 versehen. Diese Befestigungsspange 310 kann in ein distales Ende einer Schubstange (nicht gezeigt) eingesteckt werden und auf diese Weise unlösbar an dieser befestigt werden. Diese Montage ist besonders vorteilhaft für Einweginstrumente wie den vorliegenden Clipapplikator. Der vorliegende Clipapplikator wird trotz austauschbarem Clipmagazin als Einweginstrument (single use instrument) bezeichnet, da er nicht gereinigt und sterilisiert werden kann. Für eine hinreichende Reinigung müsste der Clipapplikator und somit auch das Maulteil zerlegbar sein, was mit der beschriebenen Verbindung zwischen Schieber 300 und Schubstange nicht der Fall ist. Alternativ kann die Schubstange mit einer Befestigungsspange und der Schieber mit einer entsprechenden Aufnahme bzw. Aussparung versehen sein. Auch eine lösbare Befestigung ist möglich, bei diesem Ausführungsbeispiel aber nicht vorgesehen. Mit einer lösbaren Verbindung zwischen Schieber 300 und Schubstange kann das Instrument auch als Mehrweginstrument ausgeführt werden.

Die Schubstange kann mittels eines an einem Griff vorgesehenen Betätigungshebels oder dergleichen (z.B. pneumatischer oder hydraulischer Antrieb) gegenüber dem Schaftrohr nach distal verschoben werden. Anschließend kann die Schubstange in ihre Ausgangsposition zurück gezogen werden. Diese vor und zurück Bewegung der Schubstange gegenüber dem Schaftrohr 10 bewirkt einen Öffnungs- und Schließvorgang des Maulteils.

Das Nockenträgerbauteil bzw. der Schieber 300 weist auf seiner Oberseite, die in Fig.4C gezeigt ist, drei Nocken 321, 322, 323 und auf seiner Unterseite, die in Fig. 4E gezeigt ist, drei Nocken 331, 332, 333 auf. Zudem weist der Schieber an seiner Oberseite eine Aussparung 325 und an seiner Unterseite eine Aussparung 335 auf, die sich teilweise überdecken und so einen Durchgang in dem Schieber 300 definieren.

Die obere Branche 200 weist an ihrem Kulissenelement 220 drei Kulissenbahnen 221, 222, 223 auf. An dem elastischen Fortsatz 230 der oberen Branche 200 ist im Wesentlichen gegenüber den Vorsprüngen 231, 231 ein Vorsprung bzw. ein verdickter Bereich 225 ausgebildet. In ähnlicher Weise sind an dem Kulissenelement 120 der unteren Branche 100 drei Kulissenbahnen 121, 122, 123 ausgebildet. Der elastische Fortsatz 130 der unteren Branche 100 weist zudem auch einen Vorsprung bzw. einen verdickten Bereich 125 auf, der an dem elastischen Fortsatz 130 im Wesentlichen von den Vorsprüngen 131, 132 radial abgewandt ausgebildet ist. Die beiden Branchen 100, 200 und insbesondere deren Kulissenelemente 120, 220 bilden mit dem Schieber 300 einen sandwichartigen Aufbau aus.

Die Nocken 321, 322, 323 des Schiebers 300 liegen in der genannten Reihenfolge an den Kulissenbahnen 221, 222, 223 der oberen Branche 200 an und gleiten bei einem Öffnungs- bzw. Schließvorgang an diesen entlang. Es sei hier darauf hingewiesen, dass nicht jede Nocke zu jedem Zeitpunkt eines Öffnungs- oder Schließvorgangs mit der entsprechenden Kulissenbahn in Kontakt steht. Es kann vorkommen, dass sich eine Nocke über einen gewissen Bereich eines Öffnungs- oder Schließvorgangs von der Kulissenbahn entfernt und ein Spalt zwischen der Nocke und der zugehörigen Kulissenbahn entsteht. Dieses Verhalten ist im Folgenden noch detaillierter erklärt. Hier ist lediglich aufgezeigt, welche Nocke welcher Kulissenbahn zugeordnet ist. Dies gilt selbstverständlich auch für die Kulissenbahnen 121, 122, 123 der unteren Branche 100 und die zugehörigen Nocken 331, 332, 333 des Schiebers 300.

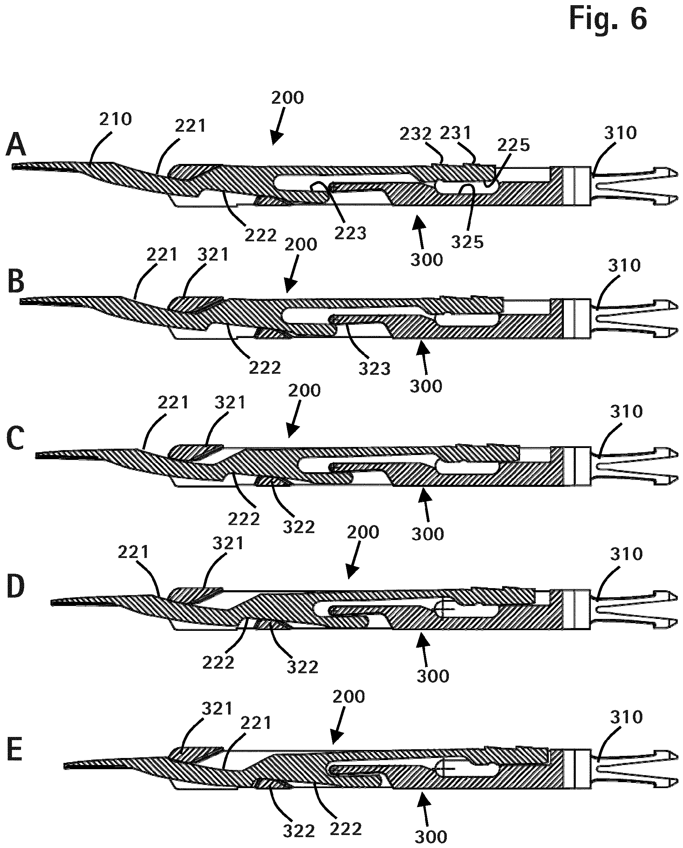

Unter Bezugnahme auf die Fig. 6 ist im Folgenden eine Montage des Maulteils 1 sowie ein Öffnungs- und Schließvorgang des Maulteils 1 am Beispiel der oberen Branche 200 beschrieben. Die Fig. 6 zeigt Draufsichten auf den Schieber 300 und die obere Branche 200, wobei ein Teil der oberen Branche 200 freigeschnitten ist, sodass man auch in der Draufsicht die Kulissenbahnen 221, 222, 223 erkennen kann. Die untere Branche 100 verhält sich entsprechend der oberen Branche, wenn auch die beiden Branchen nicht identisch sind, da die untere Branche 100 an ihrer Unterseite abgerundet ist, damit sie in dem Schaftrohr 10 Platz findet. Die untere Branche 100 ist daher für ein leichteres Verständnis der Kinematik des Systems in der Fig. 6 weggelassen. Die Fig. 6A zeigt die Montagestellung der oberen Branche 200 an dem Schieber 300, d.h. diese Stellung der oberen Branche 200 und des Schiebers 300 zueinander kann nicht mehr eingenommen werden, wenn das Maulteil einmal an einem Instrumentenschaft montiert wurde und dabei der Schieber 300 mit seiner Befestigungsspange 310 an einer Schubstange montiert wurde. In der Montagestellung ist insbesondere die Kulissenbahn 223 distal von der Nocke 323 angeordnet, sodass die obere Branche 200 in der in Fig. 6A gezeigten Stellung gegen den Uhrzeiger gedreht werden kann und somit von dem Schieber 300 entfernt werden kann.

Da bei diesem Ausführungsbeispiel die Nocken 231, 322, 323 des Schiebers 300 und die Kulissenbahnen 221, 222, 223 der oberen Branche eine Hinterschneidung ausbilden, damit sich die obere Branche nicht von dem Schieber 300 abheben kann, auch wenn keine Halteklammer vorgesehen ist, kann die obere Branche 200 nicht einfach auf den Schieber 300 aufgesetzt werden. Daher ist es wichtig, dass die obere Branche 200 in der Montagestellung gegenüber dem Schieber 300 verdreht werden kann, da nur so eine Montage der oberen Branche 200 an dem Schieber 300 möglich ist. Die obere Branche 200 wird also schräg auf den Schieber 300 aufgesetzt und dann in Uhrzeigerrichtung gedreht, bis die Kulissenbahn 221 in Anlage an die Nocke 321 gelangt und die Kulissenbahn 222 in Anlage an die Nocke 322 gelangt. Dabei bilden die Kulissenbahnen 221, 222 mit den Nocken 321, 322 Hinterschneidungen aus. Die untere Branche 100 wird mit demselben Prinzip an dem Schieber 300 montiert. Dabei gelangen die Kulissenbahnen 121, 122 mit den Nocken 331, 332 in Anlage und bilden ebenfalls Hinterschneidungen aus.

Die beiden Branchen 100, 200 und der Schieber 300 werden dann von vorn in ein Schaftrohr 10 eines Instrumentenschafts eingeschoben, in dem eine Schubstange mit einer zu der Befestigungsspange 310 passenden Aussparung angeordnet ist. Damit die Branchen 100, 200 mit dem Schieber 300 in das Schaftrohr 10 eingeschoben werden können, müssen die Vorsprünge 131, 132 und 231, 232 nach innen gedrückt werden, Damit dies möglich ist, sind in dem Schieber 300 die beiden Aussparungen 325, 335 ausgebildet. In der Montagestellungsind die Verdickungen 125, 225 der beiden Branchen 100, 200 so angeordnet, dass sie in axialer Richtung jeweils genau neben den Aussparungen 325, 335 liegen. Die elastischen Fortsätze 130, 230 der beiden Branchen 100, 200 können also elastisch nach innen verformt werden, sodass die Verdickungen 125, 225 in die Aussparungen 325, 335 eintauchen. Auf diese Weise werden die jeweils an der Außenseite vorgesehenen Vorsprünge 131, 132 und 231, 232 so weit nach innen verschoben, dass die Branchen 100, 200 in das distale Ende des Schaftrohres 10 eingeschoben werden können. Während des Einschiebens drängen die elastischen Fortsätze 130, 230 radial nach außen.

Bei fortschreitendem Einschieben der Branchen 100, 200 und des Schiebers 300 in das Schaftrohr 10 stellt die Befestigungsspange 310 dann eine Verbindung mit einer entsprechenden Aufnahme an dem Schubstab in dem Schaftrohr her. Damit ist der Schieber 300 unlösbar mit dem Schubstab verbunden. Ab diesem Zeitpunkt kann der Schieber 300 im Wesentlichen nicht mehr weiter in proximaler Richtung in das Schaftrohr 10 geschoben werden. Schiebt man nun die Branchen 100, 200 weiter in das Schaftrohr, werden die Branchen auch gegenüber dem Schieber 300 verschoben. Auf diese Weise wird eine Anlage zwischen der Nocke 323 und der Kulissenbahn 223 der oberen Branche 200 sowie eine Anlage zwischen der Nocke 333 und der Kulissenbahn 123 der unteren Branche hergestellt. In dieser Stellung sind nun die beiden Branchen 100, 200 nicht gegenüber dem Schieber 300 verdrehbar, selbst wenn kein Schaftrohr 10 vorgesehen wäre. Gleichzeitig verschieben sich die beiden Verdickungen 125, 225 der beiden Branchen 100, 200 gegenüber den Aussparungen 325, 335 des Schiebers, sodass die elastischen Fortsätze 130, 230 weiter nach außen gedrängt werden. Die beiden Branchen 100, 200 können noch einen gewissen Weg weiter in das Schaftrohr 10 geschoben werden, bis die Vorsprünge 131, 132 den Aussparungen 14 gegenüberliegen und die Vorsprünge 231, 232 den Aussparungen 15 gegenüber liegen. In dieser Position tauchen die Vorsprünge 131, 132, 231, 232 durch die Elastizität der beiden Fortsätze 130, 230 im Wesentlichen gleichzeitig in die Aussparungen 14, 15 in dem Schaftrohr 10 ein und sichern die beiden Branchen 100, 200 somit in axialer Richtung.

Bei diesem Ausführungsbeispiel ist der Abstand der beiden Vorsprünge 131, 132 minimal geringer als der Abstand der beiden Aussparungen 14. Auf diese Weise wird, wenn die untere Branche 100 mit einer Zugkraft beaufschlagt wird, d.h. wenn die untere Branche 100 nach distal gezogen wird, die Zugkraft zuerst von dem Vorsprung 131 auf die distale Wand der proximalen Aussparung 14 übertragen, was ein Moment in dem Fortsatz 130 erzeugt, welches den Vorsprung 132 zusätzlich zu der Innenwand des Schaftrohres 10 drängt. Dies bewirkt somit eine weitere Sicherung der Verbindung zwischen unterer Branche 100 und Schaftrohr 10. Dasselbe Prinzip wird auch für die obere Branche 200 verwendet, sodass auch hier eine Zugkraft zunächst von dem Vorsprung 231 auf die distale Wand der proximalen Aussparung 15 übertragen wird, bevor die distale Wand der distalen Aussparung 15 mit der distalen Wand des Vorsprungs 232 eingreift und so ebenfalls Kräfte von der oberen Branche 200 in das Schaftrohr 10 überträgt.

Das Maulteil 1 befindet sich nun in der in Fig. 6B gezeigten Grundstellung bzw. Nullstellung. Wird nun der Griff des Instruments betätigt, wird bei diesem Ausführungsbeispiel die Schubstange in dem Schaftrohr pneumatisch nach distal geschoben. Damit bewegt sich auch der Schieber 300 nach distal. Bei dieser Bewegung gleitet die Nocke 321 an der Kulissenbahn 221 entlang und verschiebt die Branche 200 bzw. deren Wirkbereich 210 in der Fig. 6 nach unten. Die Nocke 322 gleitet an der Kulissenbahn 222 entlang und ermöglicht hierdurch die vorstehend beschriebene Bewegung des Wirkbereichs 210 nach unten. Zudem gleitet die Nocke 323 an der Kulissenbahn 223 entlang und stabilisiert die Lage der Branche 200 zu dem Schieber 300. Da sich der vordere Teil der Branche 200 seitlich verschiebt, während der hintere Teil der Branche in dem Schaftrohr 10 aufgenommen ist, ist es entscheidend, dass der elastische Fortsatz 230 der oberen Branche eine solche Elastizität aufweist, dass die Verschiebung des Wirkbereichs 210 durch den elastischen Fortsatz 230 nicht verhindert oder maßgeblich beeinträchtigt wird. Das Maulteil 1 nimmt dadurch die in Fig. 6C gezeigte Stellung ein.

Bei noch weiter fortschreitendem Schließvorgang des Maulteils 1 gleiten die Nocken 321, 322 weiter an den Kulissenbahnen 221, 222 entlang, während sich die Nocke 323 von der Kulissenbahn 223 geringfügig abhebt bzw. entfernt. Die Form der Nocken 321, 322 und der Kulissenbahnen 221, 222 sind so ausgebildet, dass sich zu Beginn des Schließvorgangs der Wirkbereich 210 relativ schnell nach innen bewegt, wohingegen sich der Wirkbereich 210 zum Ende des Schließvorgangs hin langsamer nach innen bewegt. Auf diese Weise kann zu Beginn des Schließvorgangs ein relativ weiter Weg relativ schnell überwunden werden, wohingegen zum Ende des Schließvorgangs hin, wenn der Clip in dem Maulteil 1 verpresst werden muss, ein kleiner Weg zurück gelegt wird, wobei aber eine hohe Kraft auf den Clip aufgebracht werden kann. Dass zu Beginn des Schließvorgangs nur eine relativ kleine Kraft aufgebracht werden kann, stört hierbei nicht. Eine Stellung der oberen Branche 200 und des Schiebers 300 während dieser Phase des Schließvorgangs ist in Fig. 6D gezeigt.

Zum Ende des Schließvorgangs erreichen die obere Branche 200 und der Schieber 300 die in Fig. 6E gezeigte Stellung. In dieser Stellung bilden die Nocke 321 und die Kulissenbahn 221 eine maximale Anlagefläche aus, sodass in dieser Stellung auch die größte Kraft von der Nocke 321 auf die Kulissenbahn 221 ausgeübt werden kann, um das Verpressen des Clips im Wirkbereich 210 sicher zu stellen.

Während eines Öffnungsvorgangs durchlaufen die obere Branche 200 und der Schieber 300 die in den Fig. 6A bis 6E gezeigten Stellungen in umgekehrter Reihenfolge, wobei etwa in der in Fig. 6C gezeigten Stellung die Nocke 323 wieder in Kontakt mit der Kulissenbahn 232 gelangt und so den Wirkbereich 210 zwangsweise nach oben verschiebt, also das Maulteil 1 zwangsweise und geführt öffnet. Der Öffnungsvorgang hängt bei diesem Ausführungsbeispiel also nicht von einer Elastizität einer Branche oder einer elastischen Verbindung der beiden Branchen ab. Dies hat unter anderem den Vorteil, dass das Maulteil 1 nicht versehentlich z.B. beim Einführen in einen Trokar geschlossen werden kann, beispielsweise indem ein Wirkbereich einer Branche 100, 200 gegen die Innenwand des Trokars gedrückt wird und dann radial nach innen zu dem Wirkbereich der anderen Branche hin ausweicht. Sollte dies geschehen, wird der in dem Maulteil befindliche Clip gepresst und wenn sich das Maulteil 1 wieder in die Ausgangsposition aufweitet, wenn es den Trokar verlässt, dann fällt der Clip aus dem Maulteil heraus. Im vorliegenden Fall kann sich der Wirkbereich der Branchen 100, 200 nicht nach innen verschieben, wenn ein Druck von außen auf die Außenseiten der Wirkbereiche 110, 210 aufgebracht wird, da die Nocke 323 bzw. 333 an der Kulissenbahn 123 bzw. 223 anliegt und diese Kraft aufnimmt.

Die untere Branche 100, die hier nicht gezeigt und beschrieben ist, durchläuft im Wesentlichen dieselben Stellungen und unterliegt vergleichbaren Einflüssen durch den Schieber 300. Bei diesem Ausführungsbeispiel sind die Kulissenbahnen 121, 122, 123, 221, 222, 223 und die Nocken 321, 322, 323, 331, 332, 333 so gestaltet, dass sich die beiden Wirkbereiche 110, 210 der beiden Branchen 100, 200 symmetrisch zu der Mittelache des Maulteils 1 bewegen.

Dieses Ausführungsbeispiel kann zudem so modifiziert werden, dass dich das Maulteil 1 während des Schließvorgangs zunächst aufweitet, d.h. die beiden Wirkbereiche 110, 210 entfernen sich zunächst ein wenig voneinander. In Zusammenarbeit mit einem elastischen Clip, welcher leicht komprimiert in das Maulteil 1 bzw. zwischen die Wirkbereiche 110, 210 eingebracht wurde, kann auf diese Weise eine Clipöffnung bzw. Maulteilöffnung erreicht werden, die mit anderen Instrumenten mit gleichem Schaftdurchmesser nicht erreicht werden kann. Um eine vorübergehende Aufweitung des Maulteils 1 zu erreichen kann die Kulissenbahn 223 der oberen Branche 200 in der in Fig. 6C gezeigten Stellung in dem Kontaktbereich mit der Nocke 323 nach oben verschoben werden und die Kulissenbahn 221 kann in dem Kontaktbereich mit der Nocke 321 nach unten Verschoben werden. Das Maulteil 1 darf sich aber bei einer solchen Ausführungsform nicht weiter aufweiten, als der Clip mit seiner Elastizität folgen kann, da er sonst aus dem Maulteil 1 heraus fallen kann.

Der Clipspeicher bzw. das Clipmagazin dieses Ausführungsbeispiels ist bei einer Ansicht entsprechend der Fig. 5A oberhalb des sandwichartigen Aufbaus der beiden Kulissenelemente 120, 220 der beiden Branchen 100, 200 und des Schiebers 300 angeordnet. Die einzelnen Clips werden mittels eines Vorschubmechanismus, der hier nicht weiter beschrieben ist, einzeln zu den Wirkbereichen 110, 210 des Maulteils 1 vorgeschoben, wenn ein vorher im Maulteil 1 befindlicher Clip appliziert wurde.

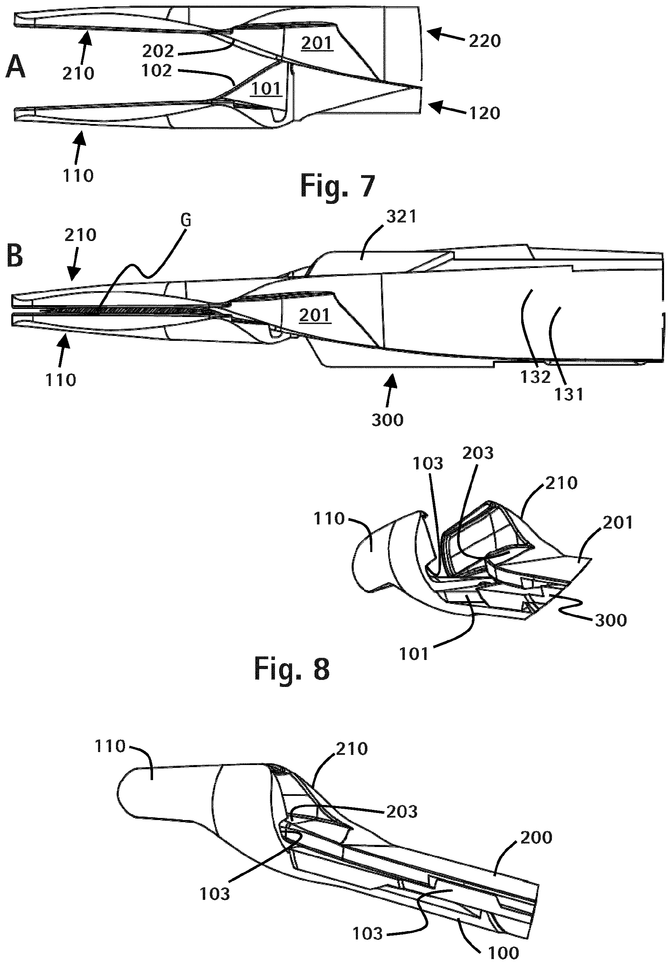

Das Maulteil 1 dieses Ausführungsbeispiels weist noch ein paar Besonderheiten auf. Eine erste Besonderheit ist im Folgenden unter Bezugnahme auf die Fig. 7 im Detail beschrieben. In dem Übergangsbereich 101, 201 zwischen dem Kulissenelement 120, 220 und dem Wirkbereich 110, 210 jeder Branche 100, 200 verändert sich jede Branche von einem in horizontaler Ebene flächigen Bauteil zu einem im Wesentlichen in vertikaler Ebene flächigen Bauteil. Damit dieser Übergangsbereich 101, 201 die Kräfte übertragen kann, die z.B. zum Verpressen eines Clips erforderlich sind, muss er in horizontaler, lateraler Richtung eine gewisse Breite aufweisen. Da die Branchen 100, 200 aber sandwichartig übereinander liegen, entsteht ein Schereffekt zwischen den beiden Übergangsbereichen 101, 201, d.h. die distalen Kanten bzw. Ränder 102, 202 des Übergangsbereich gleiten bei einem Schließvorgang des Maulteils 1 aneinander entlang. Gewebe, welches zwischen die beiden distalen Kanten 102, 202 der beiden Übergangsbereiche gelangt, kann dadurch versehentlich geschädigt oder sogar abgetrennt werden. Durch diesen Effekt kann ein Gefäß, an dem ein Clip appliziert werden soll, perforiert werden, was eine große Gefahr für den Patienten darstellt. Um diese Gefahr zu bannen sind die Übergangsbereiche 101, 201 so gestaltet, dass sich der Punkt, an dem sich die beiden distalen Kanten 201, 202 überlagern, wenn das Maulteil 1 vollständig geschlossen ist, in axialer Richtung des Maulteils 1 nicht distal vom proximalen Ende des in dem Maulteil 1 gehaltenen und verpressten Clips befindet.

Dies bedeutet, dass sich dieser Punkt in axialer Richtung auf der Höhe der Clipwurzel oder proximal davon befindet. Ist das Maulteil 1 geöffnet, befindet sich die Clipwurzel des noch unverpressten Clips somit noch vor diesem Punkt. Bringt man nun Gewebe G, beispielweise ein Gefäß, in das Maulteil 1 ein, so gelangt es bevorzugt in Anlage an die Innenseite der Clipwurzel des Clips. Der Clip verhindert auf diese Weise, dass das Gewebe G weiter nach proximal in das Maulteil 1 eingeführt werden kann. Während des Schließvorgangs verschiebt sich die Clipwurzel ein wenig nach proximal, aber niemals über den oben beschriebenen Punkt hinaus. Auf diese Weise ist sicher gestellt, dass das Gewebe G niemals durch den Schereffekt der beiden sandwichartig zusammengesetzten Kulissenelemente 120, 220 bzw. den Übergangsbereich 101, 201 der Branchen 100, 200 beschädigt wird. In der Fig. 7B ist ein Gewebe G gezeigt, welches sich in einem geschlossenen Maulteil 1 befindet. Der Übersichtlichkeit halber ist der Clip selbst in Fig. 7 nicht gezeigt. Aus Fig. 7B wird aber deutlich, dass der Clip das Eindringen des Gewebes G in das Maulteil 1 begrenzt und das Gewebe G somit schützt. Aus Sicherheitsgesichtspunkten soll sich dieser Punkt also möglichst weit proximal in dem Maulteil 1 befinden. Je weiter dieser punkt aber nach proximal verlagert wird, desto schwieriger ist es, die erforderlichen Momente auf die Wirkbereiche 110, 210 zu übertragen, um den Clip vollständig zu verpressen. Daher wird der Punkt in axialer Richtung exakt auf der Höhe der Clipwurzel im vollständig geschlossenen Zustand des Maulteils platziert. Diese Position des Punktes bewirkt eine maximale Sicherheit bei geringstmöglichen Momenten durch die Wirkbereiche 110, 120.

Eine weitere Besonderheit des Maulteils 1 des vorliegenden Ausführungsbeispiels ist im Folgenden unter Bezugnahme auf die Fig. 8 im Detail beschrieben. Diese Besonderheit dient dazu, eine ungewollte Verdrillung der beiden Wirkbereiche 110, 210 gegeneinander zu beschränken bzw. zu verhindern. Wie dies in der Fig. 8 zu erkennen ist, ist der Clip in dem Maulteil 1 abseits einer Ebne angeordnet, in der die Kraft von den Kulissenelementen 120, 220 in die Wirkbereiche 110, 210 eingeleitet wird. Eine Verdrillung der Wirkbereiche 110, 210 gegeneinander, also eine Torsion jedes Wirkbereichs zu dem zugehörigen Kulissenelement nach außen führt dazu, dass ein Winkel zwischen den Wirkbereichen 110, 210 ausgebildet wird. Dies hätte dies einen stark negativen Einfluss auf die Güte der Abklemmung eines Gefäßes durch den applizierten Clip. Die in der Fig. 8 oberen Stege eines Clips würden dann womöglich nicht vollständig gegeneinander verpresst werden und der Clip würde das Gefäß unter Umständen nicht ausreichend verschließen.

Daher verfügt der Übergangsbereich 201 der oberen Branche 200 über einen Bereich 203, der dem Übergangsbereich 101 der unteren Branche 100 zugewandt ist. Der Übergangsbereich 101 der unteren Branche 100 weist eine Vertiefung 103 auf, die dem Bereich 203 lateral gegenüberliegt. In diese Vertiefung 103 taucht der Bereich 203 während eines Schließvorgangs des Maulteils 1 eintaucht. Zu Beginn des Schließvorgangs sind die durch die Wirkbereiche 110, 210 aufgebrachten Kräfte relativ gering, sodass auch keine merkliche Verdrillung der Wirkbereiche 110, 210 eintritt. Zu Beginn des Schließvorgangs des Maulteils 1 befindet sich der Bereich 203 auch noch außerhalb der Vertiefung 103. Mit fortschreitendem Schließvorgang aber tritt der Bereich 203 in die Vertiefung 103 ein und die aneinander angepasste Form des Bereichs 203 mit der Vertiefung 103 verhindert ein Verdrillen der beiden Wirkbereiche 110, 210 gegeneinander um ihre jeweilige Längsachse. Insbesondere gegen Ende des Schließvorgangs, wo die in die Wirkbereiche 110, 210 eingeleiteten Kräfte am größten sind und die Branchen 100, 200 am stärksten dazu neigen, sich gegeneinander zu verdrillen, wirkt die Verbindung von Bereich 203 und Vertiefung 103 dem entgegen.

## Patentansprüche

1. Maulteil für ein chirurgisches Rohrschaft-Instrument mit einem Haltebauteil (10),
einer ersten Branche (100) mit einem ersten Wirkbereich (110), und einer zweiten Branche (200) mit einem zweiten Wirkbereich (210), wobei die erste Branche (100) und die zweite Branche (200) über je ein Kulissenelement (120, 220) verfügt und die Branchen (100, 200) durch das Haltebauteil (10) in axialer Richtung gehalten sind,
ein Nockenträgerbauteil (300) in dem Mauteil angeordnet ist, welches relativ zu dem Haltebauteil (10) in axialer Richtung verschiebbar ist und mindestens zwei Nocken (321, 322, 323; 331, 332, 333) trägt,
**dadurch gekennzeichnet, dass**
jedes Kulissenelement (120, 220) daran angepasst ist, bei einer relativen axialen Verschiebung zwischen dem Haltebauteil (10) und dem Nockenträgerbauteil (300) mit mindestens zwei Nocken (321, 322, 323; 331, 332, 333), die an dem Nockenträgerbauteil (300) vorgesehen sind, in Kontakt zu stehen und an diesen abzugleiten, um dabei ein Öffnen oder Schließen des Maulteils durch eine aufeinander zu oder voneinander weg gerichtete Bewegung der beiden Branchen (100, 200) zu bewirken.

2. Maulteil für ein chirurgisches Rohrschaft-Instrument nach Anspruch 1,
**dadurch gekennzeichnet, dass**
das Haltebauteil (10) einstückig mit einem Schaftbauteil des Schafts ausgebildet ist oder an diesem befestigt ist und das Nockenträgerbauteil (300) ein Schieber ist, welcher relativ zu dem Schaft axial bewegbar ist.

3. Maulteil für ein chirurgisches Rohrschaft-Instrument nach Anspruch 1 oder 2,
**dadurch gekennzeichnet, dass**
die erste Branche (100) und die zweite Branche (200) elastisch gekoppelt sind.

4. Maulteil für ein Rohrschaft-Instrument nach einem der vorangehenden Ansprüche,
**dadurch gekennzeichnet, dass**
an mindestens einem Kulissenelement (120, 220) mindestens zwei Kulissenbahnen (121, 122, 123; 221, 222, 223) ausgebildet sind.

5. Maulteil für ein chirurgisches Rohrschaft-Instrument nach einem der vorangehenden Ansprüche,
**dadurch gekennzeichnet, dass**
die erste Branche (100) und/oder die zweite Branche (100) über wenigstens einen Vorsprung (131, 132; 231, 232) verfügt, der in einen Bereich (14, 15) des Haltebauteils (10) eingreift und so eine axiale Bewegung der Branche (100, 200) gegenüber dem Haltebauteil (10) begrenzt oder verhindert.

6. Maulteil für ein chirurgisches Ins Rohrschaft-Instrument nach Anspruch 5,
**dadurch gekennzeichnet, dass**
der wenigstens eine Vorsprung (131, 132; 231, 232) an einem biegeelastischen Fortsatz (130, 230) des zugehörigen Kulissenelements (120, 220) der Branche (100, 200) vorgesehen ist und der biegeelastische Fortsatz (130, 230) den Vorsprung (131, 132; 231, 232) zu dem Haltebauteil (10) hin drängt und so einen Eingriff des wenigstens einen Vorsprungs (131, 132; 231, 232) in das Haltebauteil (10) sichert, wobei die Biegeelastizität des Fortsatzes (130, 230) bevorzugt derart eingestellt ist, dass die Bewegbarkeit und die Bewegungen des Kulissenelements (120, 220) und des Wirkbereichs (110, 210) durch den Fortsatz (130, 230) nicht beeinflusst sind.

7. Maulteil für ein chirurgisches Rohrschaft-Instrument nach einem der vorangehenden Ansprüche,
**dadurch gekennzeichnet, dass**
mindestens ein Kulissenelement (120, 220) flächig ausgebildet ist, das Nockenträgerbauteil (300) flächig ausgebildet ist und das mindestens eine Kulissenelement (120, 220) so an einer flachen Seite des Nockenträgerbauteils (300) anliegt, dass ein sandwichartiger Aufbau ausgebildet ist.

8. Maulteil für ein chirurgisches Rohrschaft-Instrument nach Anspruch 7,
**dadurch gekennzeichnet, dass**
das Nockenträgerbauteil (300) und mindestens ein Kulissenelement (120, 220) mindestens einen Bereich ausbilden, in dem eine Kulissenbahn (121, 122, 123; 221, 222, 223) und die zugehörige Nocke (321, 322, 323, 331, 332, 333) des Nockenträgerbauteils (300) eine Hinterschneidung ausbilden, sodass ein Abheben des Kulissenelements (120, 220) von dem Nockenträgerbauteil (300) verhindert ist, wobei bevorzugt mindestens ein Bereich einer Hinterschneidung über den gesamten Bewegungsbereich des Kulissenelements (120, 220) zu dem Nockenträgerbauteil (300) von einer vollständig geöffneten Stellung zu einer vollständig geschlossenen Stellung des Maulteils vorhanden ist.

9. Maulteil für ein chirurgisches Rohrschaft-Instrument nach einem der vorangehenden Ansprüche,
**dadurch gekennzeichnet, dass**
das chirurgische Instrument ein chirurgischer Clipapplikator ist und die Branchen (100, 200) des Maulteils daran angepasst sind, einen chirurgischen Clip zu halten und durch ein Schließen des Maulteils zu applizieren, wobei der chirurgische Clip als Doppelstegclip ausbildbar ist, der zwei Cliphälften aufweist, die jeweils nur an ihren beiden distalen Enden miteinander verbunden sind.

10. Maulteil für ein chirurgisches Rohrschaft-Instrument nach Anspruch 9,
**dadurch gekennzeichnet, dass**
die Wirkbereiche (110, 210) der beiden Branchen (100, 200) des Maulteils daran angepasst sind, in der vollständig geöffneten Stellung des Maulteils durch einen Clip, der im Maulteil angeordnet ist, nach außen über die laterale Position der Wirkbereiche (110, 210) der Branchen (100, 200) hinaus verschoben zu werden, welche die Wirkbereiche (110, 210) in der vollständig geöffneten Stellung des Maulteils einnehmen, wenn kein Clip im Maulteil angeordnet ist.

11. Maulteil für ein chirurgisches Rohrschaft-Instrument nach einem der Ansprüche 4 bis 10,
**dadurch gekennzeichnet, dass**
an mindestens einem Kulissenelement (120, 220) mindestens drei Kulissenbahnen (121, 122, 123; 221, 222, 223) ausgebildet sind, wobei zu jedem Zeitpunkt während des Öffnungs- und Schließvorgangs des Maulteils (1) mindestens zwei Kulissenbahnen (121, 122, 123; 221, 222, 223) an jeweils einer Nocke (321, 322, 323, 331, 332, 333) anliegen, welche an dem Nockenträger (300) vorgesehen ist.

12. Maulteil für ein chirurgisches Rohrschaft-Instrument nach Anspruch 11,
**dadurch gekennzeichnet, dass**
das Maulteil einen austauschbar anbringbaren Clipspeicher aufweist, in dem eine Vielzahl von Clips vorgesehen sind, wobei der Clipspeicher zumindest teilweise in einer Ebene angeordnet ist, die parallel zu dem sandwichartigen Aufbau des mindestens einen Kulissenelements (120, 220) mit dem Nockenträgerbauteil (300) ist, wobei die Clips zumindest teilweise in dem Clipspeicher an dem sandwichartigen Schichtaufbau vorbei zu den distalen Bereichen der Branchen (100, 200) zuführbar sind.

13. Maulteil für ein Rohrschaft-Instrument nach einem der vorangehenden Ansprüche,
**dadurch gekennzeichnet, dass**
die Wirkbereiche (110, 210) der ersten Branche (100) und der zweiten Branche (200) eine zu einer Mittelachse des Maulteils symmetrische Bewegungskurve aufweisen.

14. Maulteil für ein chirurgisches Rohrschaft-Instrument nach einem der Ansprüche 1 bis 8, 11 oder 13,
**dadurch gekennzeichnet, dass**
das chirurgische Instrument eine Schere, ein Nadelhalter, eine Klemme oder ein anderes chirurgisches Instrument ist, bei dem zwei Branchen (100, 200) aufeinander zu und/oder aneinander vorbei bewegbar sind.

## Claims

1. A jaw assembly for a surgical tubular shaft instrument comprising a supporting component (10),
a first arm (100) including a first active area (110) and
a second arm (200) having a second active area (210),
wherein each of the first arm (100) and the second arm (200) has one link element (120, 220) and the arms (100, 200) are held by the supporting component (10) in the axial direction,
a cam carrier element (300) is arranged in the jaw assembly which is axially movable relative to the supporting component (10) and carries at least two cams (321, 322, 323; 331, 332, 333),
**characterized in that**
each link element (120, 220) is designed to be in contact with at least two cams (321, 322, 323; 331, 332, 333) when there is a relative axial movement between the supporting component (10) and the cam carrier element (300), said cams being provided on the cam carrier element (300), and is designed to slide off said cams to effect an opening or closing of the jaw assembly by a movement of the two arms (100, 200) directed toward each other or away from each other.

2. The jaw assembly for a surgical tubular shaft instrument according to claim 1,
**characterized in that**
the supporting component (10) is formed integrally with a shaft component of the shaft or is fastened thereto and the cam carrier element (300) is a slide which is axially movable relative to the shaft.

3. The jaw assembly for a surgical tubular shaft instrument according to claim 1 or 2,
**characterized in that**
the first arm (100) and the second arm (200) are elastically coupled.

4. The jaw assembly for a tubular shaft instrument according to any one of the preceding claims,
**characterized in that**
at least two link paths (121, 122, 123; 221, 222, 223) are formed on at least one link element (120, 220).

5. The jaw assembly for a surgical tubular shaft instrument according to any one of the preceding claims,
**characterized in that**
the first arm (100) and/or the second arm (200) has/have at least one projection (131, 132; 231, 232) which engages in an area (14, 15) of the supporting component (10) and in this way restricts or prevents an axial movement of the arm (100, 200) relative to the supporting component (10).

6. The jaw assembly for a surgical tubular shaft instrument according to claim 5,
**characterized in that**
the at least one projection (131, 132; 231, 232) is provided on a resiliently flexible extension (130, 230) of the pertaining link element (120, 220) of the arm (100, 200) and the resiliently flexible extension (130, 230) urges the projection (131, 132; 231, 232) toward the supporting component (10) and in this way secures engagement of the at least one projection (131, 132; 231, 232) in the supporting component (10), with the flexibility of the extension (130, 230) being preferably adjusted so that the mobility and the movements of the link element (120, 220) and of the active area (110, 210) are not influenced by the extension (130, 230).

7. The jaw assembly for a surgical tubular shaft instrument according to any one of the preceding claims,
**characterized in that**
at least one link element (120, 220) is planar and the cam carrier element (300) is planar and the at least one link element (120, 220) is adjacent to a flat side of the cam carrier element (300) so that a sandwich-type structure is formed.

8. The jaw assembly for a surgical tubular shaft instrument according to claim 7,
**characterized in that**
the cam carrier element (300) and at least one link element (120, 220) form at least one area in which a link path (121, 122, 123; 221, 222, 223) and the pertaining cam (321, 322, 323, 331, 332, 333) of the cam carrier element (300) form an undercut so that the link element (120, 220) is prevented from lifting off the cam carrier element (300), wherein preferably at least one area of an undercut is provided over the entire range of movement of the link element (120, 220) to the cam carrier element (300) from a completely opened position to a completely closed position of the jaw.

9. The jaw assembly for a surgical tubular shaft instrument according to any one of the preceding claims,
**characterized in that**
the surgical instrument is a surgical clip applier and the arms (100, 200) of the jaw are adapted to hold and apply a surgical clip by closing the jaw, wherein the surgical clip can be designed as a double-webbed clip which includes two clip halves being connected to each other at their two distal ends only.

10. The jaw assembly for a surgical tubular shaft instrument according to claim 9,
**characterized in that**
the active areas (110, 210) of the two arms (100, 200) of the jaw are designed to be displaced in the completely opened position of the jaw by a clip arranged in the jaw outwards beyond the lateral position of the active areas (110, 210) of the arms (100, 200) adopted by the active areas (110, 210) in the completely opened position of the jaw when no clip is arranged in the jaw.

11. The jaw assembly for a surgical tubular shaft instrument according to any one of the claims 4 to 10,
**characterized in that**
at least three link paths (121, 122, 123; 221, 222, 223) are configured on at least one link element (120, 220), wherein at any given time during the opening and closing operation of the jaw (1) at least two link paths (121, 122, 123; 221, 222, 223) each are adjacent to one cam (321, 322, 323, 331, 332, 333) provided on the cam carrier (300).

12. The jaw assembly for a surgical tubular shaft instrument according to claim 11,
**characterized in that**
the jaw includes an exchangeably mountable clip reservoir in which a plurality of clips are provided, the clip reservoir being arranged at least partly in a plane that is in parallel to the sandwich-type structure of the at least one link element (120, 220) and the cam carrier element (300), wherein the clips can be supplied at least partly in the clip reservoir past the sandwich-type layered structure to the distal areas of the arms (100, 200).

13. The jaw assembly for a tubular shaft instrument according to any one of the preceding claims,
**characterized in that**
the active areas (110, 210) of the first arm (100) and the second arm (200) exhibit a kinematics curve symmetrical to a central axis of the jaw.

14. The jaw assembly for a surgical tubular shaft instrument according to any one of the claims 1 to 8, 11 or 13,
**characterized in that**
the surgical instrument is a pair of scissors, a needle holder, a clamp or any other surgical instrument in which two arms (100, 200) are movable toward each other and/or past each other.

## Revendications

1. Partie mâchoire pour un instrument chirurgical à tige tubulaire, avec
- une pièce de maintien (10),
- une première branche (100) avec une première zone d'action (110), et
- une deuxième branche (200) avec une deuxième zone d'action (210),
dans laquelle la première branche (100) et la deuxième branche (200) disposent chacune d'un élément coulissant (120, 220) et les branches (100, 200) sont maintenues en direction axiale par la pièce de maintien (10),
une pièce porte-cames (300) est agencée dans la partie mâchoire, est déplaçable en direction axiale par rapport à la pièce de maintien (10) et porte au moins deux cames (321, 322, 323 ; 331, 332, 333),
**caractérisée en ce que**
chaque élément coulissant (120, 220) est adapté pour, lors d'un déplacement axial relatif entre la pièce de maintien (10) et la pièce porte-cames (300), être en contact avec au moins deux cames (321, 322, 323 ; 331, 332, 333) qui sont prévues sur la pièce porte-cames (300) et glisser contre celles-ci afin de provoquer ainsi une ouverture ou fermeture de la partie mâchoire par un mouvement des deux branches (100, 200) l'une vers l'autre ou l'une loin de l'autre.

2. Partie mâchoire pour un instrument chirurgical à tige tubulaire selon la revendication 1,
**caractérisée en ce que**
la pièce de maintien (10) est réalisée d'un seul tenant avec une pièce de tige de la tige ou est fixée à celle-ci et la pièce porte-cames (300) est un coulisseau qui est déplaçable axialement par rapport à la tige.

3. Partie mâchoire pour un instrument chirurgical à tige tubulaire selon la revendication 1 ou 2,
**caractérisée en ce que**
la première branche (100) et la deuxième branche (200) sont couplées élastiquement.

4. Partie mâchoire pour un instrument chirurgical à tige tubulaire selon l'une quelconque des revendications précédentes,
**caractérisée en ce qu'**
au moins deux guides de coulisse (121, 122, 123 ; 221, 222, 223) sont réalisés sur au moins un élément coulissant (120, 220).

5. Partie mâchoire pour un instrument chirurgical à tige tubulaire selon l'une quelconque des revendications précédentes,
**caractérisée en ce que**
la première branche (100) et/ou la deuxième branche (100) disposent d'au moins une partie en saillie (131, 132 ; 231, 232) qui pénètre dans une zone (14, 15) de la pièce de maintien (10) et ainsi limite ou empêche un déplacement axial de la branche (100, 200) par rapport à la pièce de maintien (10).

6. Partie mâchoire pour un instrument chirurgical à tige tubulaire selon la revendication 5,
**caractérisée en ce que**
l'au moins une partie en saillie (131, 132 ; 231, 232) est prévue au niveau d'un prolongement (130, 230), élastique en flexion, de l'élément coulissant (120, 220) associé de la branche (100, 200) et le prolongement (130, 230) élastique en flexion pousse la partie en saillie (131, 132 ; 231, 232) vers la pièce de maintien (10) et assure ainsi une pénétration de l'au moins une partie en saillie (131, 132 ; 231, 232) dans la pièce de maintien (10), l'élasticité en flexion du prolongement (130, 230) étant alors réglée de préférence de telle sorte que la mobilité et les déplacements de l'élément coulissant (120, 220) et de la zone d'action (110, 210) ne sont pas influencés par le prolongement (130, 230).

7. Partie mâchoire pour un instrument chirurgical à tige tubulaire selon l'une quelconque des revendications précédentes,
**caractérisée en ce qu'**
au moins un élément coulissant (120, 220) est réalisé plan, la pièce porte-cames (300) est réalisée plane et l'au moins un élément coulissant (120, 220) s'appuie de telle manière contre un côté plan de la pièce porte-cames (300) qu'une structure en sandwich est réalisée.

8. Partie mâchoire pour un instrument chirurgical à tige tubulaire selon la revendication 7,
**caractérisée en ce que**
la pièce porte-cames (300) et au moins un élément coulissant (120, 220) réalisent au moins une zone dans laquelle un guide de coulisse (121, 122, 123 ; 221, 222, 223) et la came associée (321, 322, 323 ; 331, 332, 333) de la pièce porte-cames (300) réalisent une contre-dépouille de manière à empêcher un soulèvement de l'élément coulissant (120, 220) de la pièce porte-cames (300), au moins une zone d'une contre-dépouille étant présente de préférence sur toute la zone de déplacement de l'élément coulissant (120, 220) vers la pièce porte-cames (300) d'une position complètement ouverte à une position complètement fermée de la partie mâchoire.

9. Partie mâchoire pour un instrument chirurgical à tige tubulaire selon l'une quelconque des revendications précédentes,
**caractérisée en ce que**
l'instrument chirurgical est un applicateur de clips chirurgicaux et les branches (100, 200) de la partie mâchoire sont adaptées pour tenir un clip chirurgical et l'appliquer par une fermeture de la partie mâchoire, lequel clip chirurgical peut être réalisé comme clip à tige double qui comporte deux moitiés de clip qui sont reliées à chaque fois seulement au niveau de leurs deux extrémités distales.

10. Partie mâchoire pour un instrument chirurgical à tige tubulaire selon la revendication 9,
**caractérisée en ce que**
les zones d'action (110, 210) des deux branches (100, 200) de la partie mâchoire sont adaptées pour, dans la position complètement ouverte de la partie mâchoire par un clip qui est agencé dans la partie mâchoire, être déplacées vers l'extérieur au-delà de la position latérale des zones d'action (110, 210) des branches (100, 200), position que prennent les zones d'action (110, 210) dans la position complètement ouverte de la partie mâchoire lorsque aucun clip n'est agencé dans la partie mâchoire.

11. Partie mâchoire pour un instrument chirurgical à tige tubulaire selon l'une quelconque des revendications 4 à 10,
**caractérisée en ce qu'**
au moins trois guides de coulisse (121, 122, 123 ; 221, 222, 223) sont réalisés au niveau d'au moins un élément coulissant (120, 220) et, à chaque instant pendant l'opération d'ouverture et de fermeture de la partie mâchoire (1), au moins deux guides de coulisse (121, 122, 123 ; 221, 222, 223) s'appuient respectivement contre une came (321, 322, 323 ; 331, 332, 333) qui est prévue au niveau du porte-cames (300).

12. Partie mâchoire pour un instrument chirurgical à tige tubulaire selon la revendication 11,
**caractérisée en ce que**
la partie mâchoire comporte une réserve de clips dans laquelle plusieurs clips sont prévus et qui peut être placée là de manière échangeable, laquelle réserve de clips est agencée au moins en partie dans un plan qui est parallèle à la structure en sandwich de l'au moins un élément coulissant (120, 220) avec la pièce porte-cames (300), lesquels clips au moins en partie dans la réserve de clips peuvent être amenés jusqu'aux zones distales des branches (100, 200) en passant le long de la structure de couches en sandwich.

13. Partie mâchoire pour un instrument chirurgical à tige tubulaire selon l'une quelconque des revendications précédentes,
**caractérisée en ce que**
les zones d'action (110, 210) de la première branche (100) et de la deuxième branche (200) présentent une courbe de déplacement symétrique par rapport à un axe médian.

14. Partie mâchoire pour un instrument chirurgical à tige tubulaire selon l'une quelconque des revendications 1 à 8, 11 ou 13,
**caractérisée en ce que**
l'instrument chirurgical est une paire de ciseaux, un porte-aiguille, une pince ou un autre instrument chirurgical tel que deux branches (100, 200) sont déplaçables l'une vers l'autre et/ou l'une le long de l'autre.
